# EUROPÄISCHE PATENTSCHRIFT

(11) **EP 1 190 679 B1**
(45) Veröffentlichungstag und Bekanntmachung des Hinweises auf die Patenterteilung: **31.10.2007**
(21) Anmeldenummer: 01120369.2
(22) Anmeldetag: 25.08.2001
(51) Int. Cl.: A61B 19/00

(54) **Medizinisches Therapie- und/oder Diagnosegerät mit einer Positionserfassungeinrichtung**
Therapeutic and/or diagnostic medical apparatus with a position detection device
Appareil médical de diagnostic et/ou de thérapie avec un dispositif de détection de positions

(30) Priorität: 25.09.2000 DE 10047698
(43) Veröffentlichungstag der Anmeldung: 27.03.2002
(73) Patentinhaber: Carl Zeiss, 89518 Heidenheim (Brenz) (DE); Carl-Zeiss-Stiftung trading as Carl Zeiss, 89518 Heidenheim (Brenz) (DE)
(72) Erfinder: Pelzer, Martin, 89551 Zang (DE); Neubrandt, Walter, 89551 Zang (DE); Luber, Joachim, 73457 Essingen (DE); Mackevics, Arvids, 73432 Aalen (DE)

(56) Entgegenhaltungen:
- WO-A-99/38449
- DE-A- 19 640 993
- DE-A- 19 837 152

## Beschreibung

Die vorliegende Erfindung betrifft ein medizinisches Therapie- und/oder Diagnosegerät nach dem Oberbegriff von Anspruch 1.

Ein derartiges Therapie- und/oder Diagnosegerät ist z.B. aus der US 5,408,409 bekannt. Diese Druckschrift zeigt als Positionserfassungseinrichtung eine LED-Anordnung an der als chirurgisches Schneidwerkzeug ausgebildeten Funktionseinheit und in Abstand davon eine orts- und lagefeste Kameraanordnung als weitere Erfassungseinrichtung.

Die DE 196 40 993 A1 offenbart ein medizinisches Therapie- und/oder Diagnosegerät in Form eines Operationsmikroskopes mit einer Positionserfassungseinrichtung. Um ein steriles Arbeiten mit dem Gerät zu ermöglichen, wird es mit einer sterilen Schutzhülle überzogen. Diese Schutzhülle kann die Funktion der Positionserfassungseinrichtung beeinträchtigen Zur Vermeidung dieses Problemes ist deshalb bei dem medizinischen Therapie- und/oder Diagnosegerät die Positionserfassungseinrichtung abnehmbar ausgelegt. Sie kann dann, bevor die Schutzhülle über das Gerät gezogen wird, abgenommen werden. Ist dann die Schutzhülle angebracht, wird die Positionserfassungseinrichtung wieder mittels einer Ankopplungseinrichtung an dem Gerät oberhalb der Schutzhülle befestigt.

Die WO 99/38449 offenbart ein Kamerasystem für medizinische Zwecke. Mittels des Kamerasystems kann ein dreidimensionales Bild eines Patientenbereichs aufgenommen werden. Das erfasste Kamerabild wird einem Prozessor zugeführt, welcher ermöglicht, aufgrund von am Patientenbereich angeordneten Markierungselementen den Patientenbereich mit Markierungselementen einer am Kopf eines Operateurs angeordneten Bildwiedergabeeinheit und mit Markierungselementen eines chirurgischen Instruments zu referenzieren. Damit können dem Operateur in der tragbaren Bildwiedergabeeinheit beispielsweise Patientenbilder dargestellte werden, welche auf Kemspindresonarzdaten beruhen und dabei lagerichtig dem überlagert sind, was er mit seinen Augen erfasst.

Weitere gattungsgemäße Therapie- und/oder Diagnosegeräte mit einer als Operationsmikroskop ausgebildeten Funktionseinheit sind in der DE 197 51 781 A1 der Anmelderin und der DE 19837 152 A1 beschrieben.

Aufgabe der Erfindung ist es, ein insbesondere hinsichtlich der Genauigkeit der Positionserfassung und der Störungsunempfindlichkeit verbessertes gattungsgemäßes Therapie- und/oder Diagnosegerät bereitzustellen.

Diese Aufgabe wird durch die Merkmale in Anspruch 1 gelöst.

Denn durch die betriebsmäßige Orts- und Lageveränderbarkeit der Positionserfassungseinrichtung relativ zur Funktionseinheit kann durch Auswahl einer geeigneten Lage der Positionserfassungseinrichtung relativ zur Funktionseinheit die Signalstrecke unter Berücksichtigung der jeweiligen Operationssituation zur Vermeidung von z.B. durch Operationspersonal hervorgerufenen Abschattungen optimiert werden.

Vorteilhafte Ausführungsformen der Erfindung sind Gegenstand der abhängigen Ansprüche.

Bei einer Ausführungsform umfasst der Positionserfassungsarm eine Sendeeinrichtung mit aktiven Markern, z.B. mindestens drei räumlich verteilt angeordneten Sendeelementen, wie LED's oder Ultraschallsender. Selbstverständlich kann die Positionserfassungseinrichtung aber auch passive Marker, z.B. reflektierende Flächen, aufweisen, welche von der weiteren Erfassungseinrichtung erfassbar sind.

Die folgenden, sich auf die beigefügten Zeichnungen beziehende Beschreibung eines Ausführungsbeispiels dient dem Verständnis der Erfindung.

Es zeigen:
- Figur 1: eine schematische Darstellung eines Operationsmikroskops als eine Ausführungsform der Erfindung; und
- Figur 2: eine perspektivische Darstellung des Operationsmikroskopes von Figur 1 mit einem Pointer.

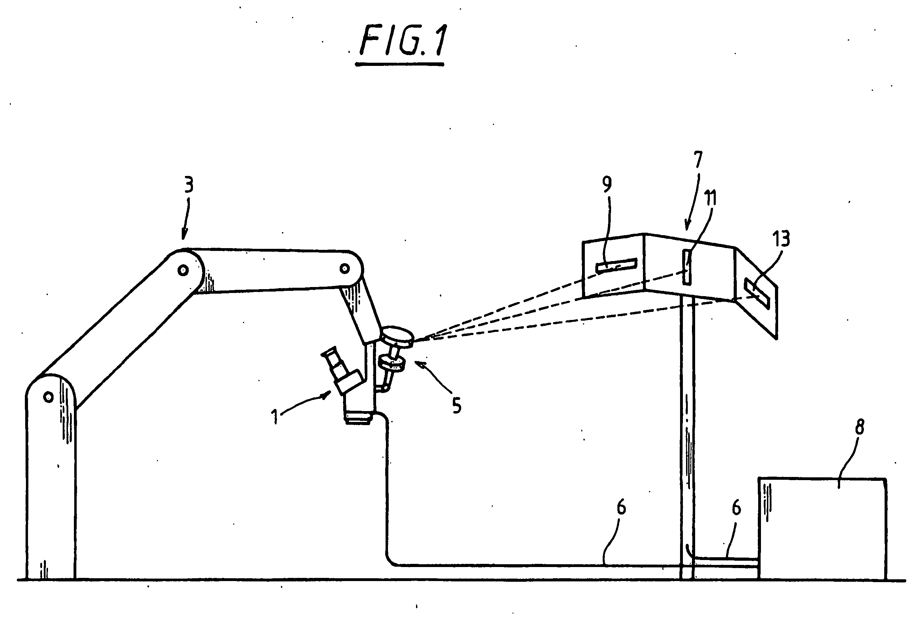

Das in Figur 1 schematisch dargestellte medizinische Therapie- und/oder Diagnosegerät 1 ist ein Operationsmikroskop, welches mittels eines gelenkigen Stativs 3 verschieb- und verschwenkbar ist. Zur Erfassung der jeweiligen Lage des Operationsmikroskops 1 ist eine Positions- und Lageerfassungsanordnung mit einer Positionserfassungseinrichtung 5 am Operationsmikroskop 1 und einer orts- und lagefesten weiteren Erfassungseinrichtung 7 vorgesehen. Die Positionserfassungseinrichtung 5 und die weitere Erfassungseinrichtung 7 sind über schematisch angedeutete Leitungen 6 mit einer Positions- und Lageauswerteeinheit 8 verbunden. Da die der Erfindungsgattung zugrunde liegende Positions- und Lageerfassung auf einer direkten Signalübermittlung, z.B. durch Licht oder Ultraschall, zwischen der ersten Erfassungseinrichtung 5 und der zweiten Erfassungseinrichtung 7 beruht, ist die in dieser Ausführungsform als Anordnung dreier Zeilenkameras 9, 11, 13 ausgebildete weitere Erfassungseinrichtung 7 möglichst derart anzuordnen, daß die Signalstrecke zu der als Sendeeinrichtung ausgebildeten ersten Erfassungseinrichtung 5 frei von Hindernissen ist.

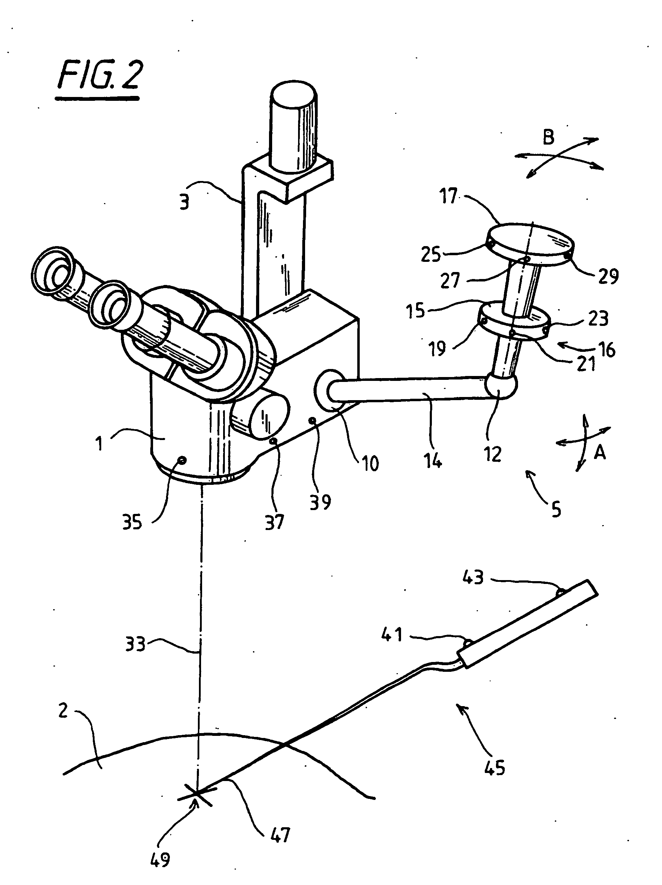

In Figur 2 sind die erfindungsgemäß als Positionserfassungsarm 5 ausgebildete Positionserfassungseinrichtung und das Operationsmikroskop 1 zusammen mit einem Pointer 45 und einem Beobachtungsobjekt 2 perspektivisch dargestellt

Dabei ist zu erkennen, daß der Positionserfassungsarm 5 ein längsgestrecktes Auslegerelement 14 und ein Positionserfassungteil 16 mit einem Radialvorsprung 15 und einem von diesem in Abstand angeordneten weiteren Radialvorsprung 17 aufweist.

Von den um den Umfangsrand der Radialvorsprungs 15 bzw. 17 angeordneten Sendeelementen, in diesem Fall LED's, sind die Sendeelemente 19, 21 und 23 bzw. 25, 27 und 29 zu erkennen.

Das Auslegerelement 14 ist über ein Kugelgelenk 10 an dem Operationsmikroskop 1 angebracht und deshalb in Richtung der Doppelpfeile A verschwenkbar. Am Auslegerelement 14 ist über ein weiteres Kugelgelenk 12 das Positionserfassungteil 16 befestigt, welches dadurch relativ zum Auslegerelement 14 in Richtung Doppelpfeile B verschwenkbar ist.

Alternativ oder zusätzlich kann der Positionserfassungsarm 5 relativ zum Operationsmikroskop 1 auch verschiebbar ausgebildet sein.

Die Positions- und Lageauswerteeinheit 8 muß dabei stets über die jeweilige Relativlage des Positionserfassungsarms 5 zum Operationsmikroskop 1 informiert sein. Dies ist z. B. durch Drehwinkelsensoren erreichbar, die in den Gelenken 10 und 12 angeordnet sind und eine Signalleitung zur Positions- und Lageauswerteeinheit 8 aufweisen.

Es kann aber auch das folgende Verfahren mit einem aus der US 5,383,454 bekannten Pointer 45 durchgeführt werden.

Der handgehaltene Pointer 45 weist zwei Sendeelemente 41 und 43 auf, welche zusammen mit der Pointerspitze 47 auf einer Geraden liegen und von der weiteren Erfassungseinrichtung 7 orts- und lagemäßig erfaßbar sind. Dabei ist der Abstand der Pointerspitze 47 von den Sendeelemente 41 und 43 der Positions- und Lageauswerteeinheit 8 bekannt, weshalb aus der Lage der Sendeelemente 41 und 43 die Lage der Pointerspitze bestimmbar ist.

Mit der Pointerspitze 47 werden Antastpunkte 35, 37 und 39 am Operationsmikroskop 1 angetastet und durch Aktivieren der Sendeelemente 41 und 43 koordinatenmäßig bestimmt. Durch Aktivieren der Sendeelemente 19, 21, 23, 25, 27 und 29 des Positionserfassungsarms 5 wird dann die Lage des Positionserfassungsarms 5 koordinatenmäßig bestimmt. Daraus kann die Positions- und Lageauswerteeinheit 8 dann die Relativlage des Positionserfassungsarm 5 zum Operationsmikroskop berechnen.

Da die Lagebeziehungen der Antastpunkte 35, 37 und 39 zur optischen Achse 33 des Operationsmikroskops 1 fixiert und bekannt sind, kann daraus insbesondere zusammen mit den Daten über die jeweilige Schnittweite des Operationsmikroskops 1 die Lage- und Ortsbeziehung des Positionserfassungsarms 5 zum Fokus 49 des Operationsmikroskops 1 bestimmt werden.

Ergänzend oder alternativ kann aber mit dem Pointer 45, wie dargestellt, auch direkt der durch das Operationsmikroskop 1 z. B. anhand eines Strichkreuzes erkennbare Fokus 49 angetastet und somit die Lage- und Ortsbeziehung des Positionserfassungsarms 5 zum Fokus 49 des Operationsmikroskops 1 mit erhöhter Genauigkeit und/oder ohne ein Antasten der Antastpunkte 35, 37 und 39 am Operationsmikroskop 1 bestimmt werden.

## Patentansprüche

1. Medizinisches Therapie- und/oder Diagnosegerät
- mit einer Funktionseinheit (1) und
- mit einer Positionserfassungseinrichtung (5) welche dazu bestimmt ist, mit einer durch eine Signalstrecke von der Positionserfassungseinrichtung (5) getrennt sowie orts- und lagefest anzuordnenden, weiteren Erfassungseinrichtung (7) zusammenzuwirken, um bei Aktivierung die Position des medizinischen Therapie- und/oder Diagnosegeräts zu erfassen,
**dadurch gekennzeichnet, dass**
- Mittel zum Erfassen der jeweiligen Relativlage der Positionserfassungseinrichtung (5) zu der Funktionseinheit (1) des medizinischen Therapie- und/oder Diagnosegeräts vorgesehen sind, so dass die aktivierte Positionserfassungseinrichtung (5) relativ zur Funktionseinheit (1) im laufenden Betrieb orts- und lageveränderbar ist.

2. Therapie- und/oder Diagnosegerät nach Anspruch 1, wobei die Funktionseinheit (1) ein Operationsmikroskop ist.

3. Therapie- und/oder Diagnosegerät nach Anspruch 1 oder 2, wobei die Positionserfassungseinrichtung als ein sich von der Funktionseinheit (1) weg erstreckender Positionserfassungsarm (5) ausgebildet ist,

4. Therapie- und/oder Diagnosegerät nach Anspruch 3, wobei der Positionserfassungsarm (5) ein über ein Kugelgelenk (10) mit der Funktionseinheit (1) verbundenes Auslegerelement (14) aufweist

5. Therapie- und/oder Diagnosegerät nach Anspruch 3, wobei der Positionserfassungsarm (5) einen über ein Kugelgelenk (12) mit dem Auslegerelement (14) verbundenen Positionserfassungsteil (16) aufweist, welcher in Abstand von der Funktionseinheit (1) angeordnet ist.

6. Therapie- und/oder Diagnosegerät nach einem der Ansprüche 1 bis 5, wobei die Funktionseinheit (1) Antastpunkte (35, 37, 39) aufweist.

7. Therapie- und/oder Diagnosegerät nach einem der Ansprüche 3 bis 6, wobei der Positionserfassungsarm (5) eine Sendeeinrichtung mit mindestens drei räumlich verteilt angeordneten Sendeelementen (19, 21, 23, 25, 27, 29) umfaßt.

## Claims

1. Medical therapy and/or diagnosis operation
- having a functional unit (1) and
- having a position detection device which is intended to cooperate with a further detection device (7) to be arranged fixed in orientation and position and separated from the position detection device (5) by a signal path, in order to detect the position of the medical therapy and/or diagnosis apparatus upon activation,
**characterized in that**
- means are provided for detecting the respective orientation of the position detection device (5) relative to the functional unit (1) of the medical therapy and/or diagnosis apparatus, so that the activated position detection device (5) can have its position and orientation changed relative to the functional unit (1) in the course of operation.

2. Therapy and/or diagnosis device according to Claim 1, wherein the functional unit (1) is an operation microscope.

3. Therapy and/or diagnosis device according to Claim 1 or 2, wherein the position detection device is designed as a position detection arm (5) extending away from the functional unit (1).

4. Therapy and/or diagnosis device according to Claim 3, wherein the position detection arm (5) has a boom element (14) connected to the functional unit (1) via a ball joint (10).

5. Therapy and/or diagnosis device according to Claim 3, wherein the position detection arm (5) has a position detection part (16) which is connected to the boom element (14) via a ball joint (10) and is arranged at a distance from the functional unit (1).

6. Therapy and/or diagnosis device according to one of Claims 1 to 5, wherein the functional unit (1) has sensing points (35, 37, 39).

7. Therapy and/or diagnosis device according to one of Claims 2 to 6, wherein the position detection arm (5) comprises a transmitter device having at least three transmitter elements (19, 21, 23, 25, 27, 29) arranged spatially distributed.

## Revendications

1. Appareil de thérapie et/ou de diagnostic médical, qui comprend :
- une unité fonctionnelle (1) et
- un dispositif (5) de détection de position destiné à coopérer avec un autre dispositif de détection (7) séparé par un parcours de signaux du dispositif (5) de détection de position et immobilisé en une position fixe pour, lors de l'activation, détecter la position de l'appareil médical de thérapie et/ou de diagnostic,
**caractérisé en ce que**
- il présente des moyens de détection de la position relative particulière du dispositif (5) de détection de position par rapport à l'unité fonctionnelle (1) de l'appareil médical de thérapie et/ou de diagnostic, de telle sorte que la position et l'immobilisation du dispositif (5) de détection de position activé par rapport à l'unité fonctionnelle (1) puissent être modifiées en continu.

2. Appareil de thérapie et/ou de diagnostic selon la revendication 1, dans lequel l'unité fonctionnelle (1) est un microscope opératoire.

3. Appareil de thérapie et/ou de diagnostic selon les revendications 1 ou 2, dans lequel le dispositif de détection de position est configuré comme bras (5) de détection de position qui déborde de l'unité fonctionnelle (1).

4. Appareil de thérapie et/ou de diagnostic selon la revendication 3, dans lequel le bras (5) de détection de position présente un élément de bras (14) relié à l'unité fonctionnelle (1) par une articulation sphérique (10).

5. Appareil de thérapie et/ou de diagnostic selon la revendication 3, dans lequel le bras (5) de détection de position présente une partie (16) de détection de position reliée à l'élément de bras (14) par une articulation sphérique (12) et disposée à distance de l'unité fonctionnelle (1).

6. Appareil de thérapie et/ou de diagnostic selon l'une des revendications 1 à 5, dans lequel l'unité fonctionnelle (1) présente des points de palpage (35, 37, 39).

7. Appareil de thérapie et/ou de diagnostic selon l'une des revendications 3 à 6, dans lequel le bras (5) de détection de position comprend un dispositif émetteur qui présente au moins trois éléments émetteurs (19, 21, 23, 25, 27, 29) répartis dans l'espace.
